# FASCICULE DE BREVET EUROPEEN

(11) **EP 0 714 888 B1**
(45) Date de publication et mention de la délivrance du brevet: **04.02.1998**
(21) Numéro de dépôt: 95402601.9
(22) Date de dépôt: 20.11.1995
(51) Int. Cl.: C07C 303/16, C07C 309/04

(54) **Préparation d'acides alcanésulfoniques par photooxydation de dérivés sulfurés**
Herstellung von Alkansulfonsäuren durch Photooxidation von Schwefelderivaten
Preparation of alkanesulphonic acids by photo-oxidation of sulphur derivatives

(30) Priorité: 29.11.1994 FR 9414279
(43) Date de publication de la demande: 05.06.1996
(73) Titulaire: ELF ATOCHEM S.A., 92800 Puteaux (FR)
(72) Inventeur: Banchereau, Evelyne, Chez M.C. Auriel, Rue Salvador Allende, F-64000 Pau (FR); Lacombe, Sylvie, F-64230 Artiguelouve (FR); Ollivier, Jean, F-64260 Arudy (FR)
(74) Mandataire: Leboulenger, Jean

(56) Documents cités:
- EP-A- 0 627 414
- US-A- 2 697 722
- US-A- 3 392 095
- JOURNAL OF ORGANIC CHEMISTRY, vol. 37, no. 22, 3 Novembre 1972 WASHINGTON, DC, US, pages 3516-3520, R.W. MURRAY, ET AL.: 'Photosensitised oxidation of dialkyl disulphides'
- TETRAHEDRON LETTERS, vol. 35, no. 27, 4 Juillet 1994 OXFORD, GB, pages 4723-4726, E.L. CLENNAN, ET AL.: 'Photooxidations of sulphenic acid derivatives 2. A remarkable solvent effect on the reactions of singlet oxygen with disulphides'

## Description

La présente invention concerne la synthèse d'acides alcanesulfoniques et a plus particulièrement pour objet la préparation des acides alcanesulfoniques en C₁ à C₄ à partir des dérivés sulfurés correspondants.

Les acides alcanesulfoniques et leurs sels ont de nombreuses applications industrielles, notamment comme détergents, émulsifiants, catalyseurs d'estérification, durcisseurs pour certaines résines.

Industriellement, les acides alcanesulfoniques sont le plus souvent fabriqués à partir des alcanes par sulfoxydation ou par sulfochloration. Ces deux voies de synthèse présentent cependant l'inconvénient de conduire à la formation de sous-produits sulfonés sur les différents atomes de carbone de la chaîne hydrocarbonée. D'autre part, l'hydrolyse des alcanesulfochlorures conduit généralement à des acides alcanesulfoniques plus ou moins colorés, et à la production non désirée d'acide chlorhydrique.

Ces dernières années, de nombreux travaux ont été consacrés à la photochimie atmosphérique de composés organiques sulfurés (thiols, sulfures, disulfures) qui sont, à l'état de traces, des constituants atmosphériques d'origine biogénique ou, plus souvent, des polluants issus des technologies de production d'énergie. La plupart de ces études photochimiques ont été effectuées en phase gazeuse sous faible pression (≤ 1 atm) en présence d'air, d'oxygène, d'un gaz éluant et parfois d'oxydes d'azote. Tous les résultats obtenus mentionnent la formation de dioxyde de soufre, d'acides sulfonique et sulfurique, ainsi que d'autres produits variant selon les conditions mises en oeuvre.

Les recherches sur la photooxydation en solution de composés organiques sulfurés ont été presque uniquement consacrées à la photooxydation des sulfures en présence de photosensibilisants pour la préparation de sulfoxydes et de sulfones. Deux articles, l'un de R.W. MURRAY et al. (J. Org. Chem., 37, 1972, pp.3516-3520) et l'autre de E.L. CLENNAN et al. (Tetrahedron Lett. 35, 1994, pp.4723-6) concernent la formation de thiosulfinates et de thiosulfonates par photooxydation des disulfures en présence de photosensibilisants.

Le brevet US 3 392 095 décrit un procédé pour transformer un disulfure d'alkyle R'-S-S-R'' en acide sulfonique R'-SO₃H ou R''-SO₃H par oxydation par l'oxygène en présence d'une quantité catalytique d'un oxyde d'azote choisi parmi NO, NO₂, N₂O₃, N₂O₄, N₂O₅, d'un solvant choisi parmi les hydrocarbures chlorés et les hydrocarbures aromatiques, et d'environ 0,001 à environ 25% en poids d'eau basé sur l'oxyde d'azote, la réaction étant initiée par de la lumière actinique.

Il a maintenant été trouvé que d'excellents rendements en acides alcanesulfoniques peuvent être obtenus à partir des thiols, sulfures et disulfures, par photooxydation en présence d'oxygène sans catalyseur si l'on opère en solution dans l'acétonitrile aqueux ou alcoolique, avec une source lumineuse irradiant entre 200 et 400 nm.

L'invention a donc pour objet un procédé de préparation d'un acide alcanesulfonique R-SO₃H à partir d'un dérivé sulfuré R-S-X correspondant, X représentant un atome d'hydrogène, un radical alkyle R ou un groupe alkylthio -SR, caractérisé en ce qu'il consiste à soumettre une solution du dérivé sulfuré dans l'acétonitrile aqueux ou alcoolique, en présence d'oxygène, à une irradiation par des rayonnements lumineux de longueur d'onde comprise entre 200 et 400 nm.

Le procédé selon l'invention s'applique plus particulièrement à la synthèse des acides alcanesulfoniques dont la chaîne hydrocarbonée, linéaire ou ramifiée, peut contenir de 1 à 4 atomes de carbone. Comme exemples de dérivés sulfurés à utiliser comme produits de départ, on peut mentionner, à titre non limitatif, le diméthyl sulfure, le diméthyl disulfure, le diéthyl disulfure, le dipropyl disulfure, le n.butyl thiol, le di-n.butyl sulfure, le di-n.butyl disulfure, le di-tert.butyl disulfure.

La concentration en dérivé sulfuré dans la solution réactionnelle de départ peut généralement aller de 0,01 à 5 mole/litre, mais elle est de préférence comprise entre 0,05 et 0,5 mole/litre.

La quantité d'eau ou d'alcool dans la solution réactionnelle peut varier dans de larges limites. Elle peut aller jusqu'à 100 moles par mole de dérivé sulfuré, mais est de préférence comprise entre 1 et 50 moles par mole de dérivé sulfuré. Comme alcool on utilise de préférence un alcool en C₁ à C₄ et, plus particulièrement, le méthanol.

L'oxygène, nécessaire à la réaction, peut être fourni sous forme pure ou diluée par un gaz inerte tel que, par exemple, l'azote. L'oxygène est de préférence introduit progressivement au sein de la solution réactionnelle. La quantité totale d'oxygène nécessaire est d'au moins 2 moles par mole de thiol ou de sulfure présent dans la solution initiale, et d'au moins 4 moles par mole de disulfure. Il est préférable d'opérer avec un excès d'oxygène d'au moins 50 %.

La photooxydation selon l'invention peut être menée à une température comprise entre -20°C et le point d'ébullition du mélange réactionnel, mais on opère de préférence entre 5 et 45°C. L'opération est avantageusement réalisée à la pression atmosphérique, mais on ne sortirait pas du cadre de la présente invention en travaillant sous pression.

La procédé selon l'invention peut être mis en oeuvre de manière discontinue ou continue, dans tout réacteur photochimique, par exemple dans un réacteur à immersion ou à film tombant, équipé d'une ou plusieurs lampes à vapeur de mercure à basse, moyenne ou haute pression, ou de lampes excimères émettant dans l'ultraviolet.

Les exemples suivants illustrent l'invention, sans la limiter.

### EXEMPLE 1

On utilise un réacteur photochimique équipé d'un porte-lampe central à immersion. La source lumineuse est une lampe Hanovia, moyenne pression de mercure, 450 W (référence 679 A) et le porte-lampe utilisé est en quartz. Avec ce porte-lampe, le volume de liquide dans le réacteur est de 250 ml et le trajet optique de 6,5 mm. Le réacteur et le porte-lampe sont thermostatés par une circulation d'eau courante et l'oxygène est introduit à pression atmosphérique par le bas du réacteur à travers un fritté (porosité 4). Pour minimiser l'entraînement des produits volatils, l'effluent gazeux en sortie du réacteur passe à travers un réfrigérant refroidi à l'eau ; il est ensuite admis dans un barboteur à soude et enfin dirigé vers une torche.

Dans le réacteur, on a introduit 250 ml d'une solution 0,2 M en diméthyldisulfure (soit 0,05 mole de DMDS) dans l'acétonitrile contenant 27 ml d'eau. La solution a été laissée sous bullage d'oxygène (débit : 2,8 litres/heure) pendant 5 à 10 minutes à 15°C, puis on a allumé la lampe et, tout en maintenant le même débit d'oxygène, on a irradié pendant 2 heures et 45 minutes à 35-40°C.

Après cette période, la solution était claire et homogène et on ne remarquait la présence d'aucun précipité. Après évaporation du solvant, on a obtenu 17,3 g d'un liquide incolore dont l'analyse par potentiométrie, gravimétrie et RMN¹H indiquait une conversion quasi-totale (98 %) du DMDS avec un rendement de 62 % en acide méthanesulfonique (AMS) et de 19 % en acide sulfurique.

### EXEMPLE 2 (Comparatif)

On a répété l'exemple 1, mais en utilisant 250 ml d'une solution 0,2 M en DMDS dans l'acétonitrile anhydre et en poursuivant l'irradiation pendant 4 heures et 15 minutes.

Après évaporation du solvant, on a obtenu 7,7 g d'un produit brut marron et inhomogène dont l'analyse, après reprise à l'eau et extraction au chloroforme, indiquait une conversion totale (100 %) du DMDS avec un rendement de 44 % en acide méthanesulfonique et de 1,2 % en acide sulfurique.

### EXEMPLES 3 A 10

On a répété l'exemple 1, mais en faisant varier la quantité d'eau. Les résultats obtenus sont rassemblés dans le tableau I suivant.

**TABLEAU I**

| **EXEMPLE** | **3** | **4** | **5** | **6** | **7** | **8** | **9** | **10** |
|---|---|---|---|---|---|---|---|---|
| Quantité d'eau (ml) | 0,9 | 1,8 | 1,8 | 2,7 | 9,9 | 18 | 57,6 | 90 |
| Durée d'irradiation (heures et minutes) | 3h | 1h30 | 3h30 | 3h30 | 3h | 2h45 | 2h40 | 3h |
| Taux de conversion du DMDS (%) | 98 | 60 | 99 | 99 | 99 | 98 | 100 | 100 |
| Rendement en AMS (%) | 44 | 55* | 57 | 55 | 66 | 70 | 62 | 44,1 |
| Rendement en acide sulfurique (%) | 4 | 3,4* | 10 | 14 | 19 | 18 | 21 | 21,5 |

| | | | | | | | | |
|---|---|---|---|---|---|---|---|---|
| * Rendement par rapport au DMDS consommé | | | | | | | | |

### EXEMPLES 11 A 14

On a opéré comme à l'exemple 1 mais en remplaçant le DMDS par la même quantité molaire d'autres dialkyl disulfures R-SS-R et en utilisant seulement 1,8 ml d'eau (2 moles par mole de disulfure). Les résultats obtenus sont rassemblés dans le tableau suivant.

**TABLEAU II**

| **EXEMPLE** | **11** | **12** | **13** | **14** |
|---|---|---|---|---|
| Radical alkyl R | C₂H₅ | n-C₃H₇ | n-C₄H₉ | t-C₄H₉ |
| Durée d'irradiation (heures et minutes) | 4h30 | 4h | 4h30 | 6h |
| Taux de conversion du disulfure (%) | 100* | 100** | 100* | 100 |
| Rendement en acide R-SO₃H (%) | 49 | 46 | 56 | 50 |
| Rendement en acide sulfurique (%) | 17,1 | 16 | 13 | 16 |

| | | | | |
|---|---|---|---|---|
| * après 3h30 | | | | |
| ** après 3h | | | | |

### EXEMPLES 15 ET 16

On a opéré comme à l'exemple 1 mais en remplaçant le DMDS par la même quantité molaire de di-n.butyl sulfure (Exemple 15) ou de n.butylthiol (Exemple 16) et en utilisant seulement 1,8 ml d'eau (2 moles par mole de dérivé sulfuré).

Les résultats obtenus sont rassemblés dans le tableau III suivant.

**TABLEAU III**

| **EXEMPLE** | **15** | **16** |
|---|---|---|
| Durée d'irradiation (heures) | 3h | 3h |
| Taux de conversion du dérivé sulfuré(%) | 100 (à 2h) | 100 (à 2h) |
| Rendement en acide n.butylsulfonique | 50 | 63 |
| Rendement en acide sulfurique (%) | 10,4 | 21 |

## Revendications

1. Procédé de préparation d'un acide alcanesulfonique R-SO₃H à partir d'un dérivé sulfuré R-S-X correspondant, X représentant un atome d'hydrogène, un radical alkyle R ou un groupe alkylthio -SR, caractérisé en ce qu'il consiste à soumettre une solution du dérivé sulfuré dans l'acétonitrile aqueux ou alcoolique, en présence d'oxygène, à une irradiation par des rayonnements lumineux de longueur d'onde comprise entre 200 et 400 nm.

2. Procédé selon la revendication 1, dans lequel le ou les radicaux alkyle R du dérivé sulfuré sont des radicaux alkyle, linéaires ou ramifiés, contenant de 1 à 4 atomes de carbone.

3. Procédé selon la revendication 1 ou 2, dans lequel la concentration en dérivé sulfuré dans la solution réactionnelle de départ peut aller de 0,01 à 5 mole/litre et est, de préférence, comprise entre 0,05 et 0,5 mole/litre.

4. Procédé selon l'une des revendications 1 à 3, dans lequel la quantité d'eau ou d'alcool dans la solution réactionnelle est comprise entre 1 et 100 moles par mole de dérivé sulfuré, de préférence entre 1 et 50 moles.

5. Procédé selon l'une des revendications 1 à 4, dans lequel on opère à une température comprise entre -20°C et le point d'ébullition du mélange réactionnel, de préférence entre 5 et 45°C.

6. Procédé selon l'une des revendications 1 à 5, dans lequel on opère à pression atmosphérique ou sous pression.

7. Application du procédé selon l'une des revendications 1 à 6 à la synthèse de l'acide méthanesulfonique à partir du diméthyldisulfure.

## Claims

1. Process for the preparation of an alkanesulphonic acid R-SO₃H from a corresponding sulphur-containing derivative R-S-X, X representing a hydrogen atom, an alkyl radical R or an alkylthio group -SR, characterized in that it consists in submitting a solution of the sulphur-containing derivative in aqueous or alcoholic acetonitrile, in the presence of oxygen, to an irradiation by light rays of wavelength between 200 and 400 nm.

2. Process according to Claim 1, in which the alkyl radical or radicals R of the sulphur-containing derivatives are linear or branched alkyl radicals containing from 1 to 4 carbon atoms.

3. Process according to Claim 1 or 2, in which the concentration of sulphur-containing derivative in the starting reaction solution may range from 0.01 to 5 mol/litre and is preferably between 0.05 and 0.5 mol/litre.

4. Process according to one of Claims 1 to 3, in which the amount of water or alcohol in the reaction solution is between 1 and 100 mol per mole of sulphur-containing derivative and preferably between 1 and 50 mol.

5. Process according to one of Claims 1 to 4, in which the procedure is carried out at a temperature between -20°C and the boiling point of the reaction mixture and preferably between 5 and 45°C.

6. Process according to one of Claims 1 to 5, in which the procedure is carried out at atmospheric pressure or under pressure.

7. Application of the process according to one of Claims 1 to 6 to the synthesis of methanesulphonic acid from dimethyl disulphide.

## Patentansprüche

1. Verfahren zur Herstellung einer Alkansulfonsäure R-SO₃H ausgehend von einem entsprechenden Schwefelderivat R-S-X, wobei X ein Wasserstoffatom, einen Alkylrest R oder eine Alkylthiogruppe -SR darstellt, dadurch gekennzeichnet, daß man bei dem Verfahren eine Lösung des Schwefelderivates in wäßrigem oder alkoholischem Acetonitril in Gegenwart von Sauerstoff einer Bestrahlung mit Lichtstrahlen einer Wellenlänge zwischen 200 und 400 nm unterwirft.

2. Verfahren nach Anspruch 1, bei dem der oder die Alkylreste R des Schwefelderivates lineare oder verzweigte Alkylreste mit 1 bis 4 Kohlenstoffatomen sind.

3. Verfahren nach Anspruch 1 oder 2, bei dem die Konzentration an Schwefelderivat in der Ausgangsreaktionslösung von 0,01 bis 5 mol/l gehen kann und vorzugsweise 0,05 bis 0,5 mol/l beträgt.

4. Verfahren nach einem der Ansprüche 1 bis 3, bei dem die Menge an Wasser oder Alkohol in der Reaktionslösung zwischen 1 und 100 mol pro Mol des Schwefelderivates, vorzugsweise zwischen 1 und 50 mol, beträgt.

5. Verfahren nach einem der Ansprüche 1 bis 4, bei den, man bei einer Temperatur zwischen -20 °C und dem Siedepunkt der Reaktionsmischung, vorzugsweise zwischen 5 und 45°C, verfährt.

6. Verfahren nach einem der Ansprüche 1 bis 5, bei dem man bei Atmosphärendruck oder unter Druck verfährt.

7. Anwendung des Verfahrens nach einem der Ansprüche 1 bis 6 zur Synthese von Methansulfonsäure ausgehend von Dimethyldisulfid.
